Europäisches Patentamt

European Patent Office

Office européen des brevets

(19)

(11) Veröffentlichungsnummer: **0 021 405**
**A1**

## (12) EUROPÄISCHE PATENTANMELDUNG

(21) Anmeldenummer: 80103563.5

(22) Anmeldetag: 24.06.80

(51) Int. Cl.³: **A 61 M 5/16**

(30) Priorität: 26.06.79 DE 7918241 U

(43) Veröffentlichungstag der Anmeldung: 07.01.81
Patentblatt 81/1

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Braun, Bernd, Dr., Tränkelücke 1, D-3508 Melsungen (DE)**

(74) Vertreter: **von Kreisler, Alek et al, Deichmannhaus am Hauptbahnhof, D-5000 Köln 1 (DE)**

(54) **Tropfkammer mit verbesserter Sitzfestigkeit des Tropfkammerdorns.**

(57) Die Erfindung betrifft eine Tropfkammer für ein Infusions- und Transfusionsgerät, die einen Einstechdorn zum Einstechen in den gummiartigen, elastischen Verschluß-Stopfen oder die Verschlußscheibe des Infusionsbehälters aufweist, der im Bereich seines spitz zulaufenden Endes mit einem Einflußloch versehen ist, auf der Außenfläche des Einstechdorns (1) sind auf einem an das Einflußloch anschließenden Abschnitt (3) in Achsrichtung parallel aufeinander folgende umfangsmäßig verlaufende Profilierungen ausgebildet.

COMPLETE DOCUMENT

Infusions- und Transfusionsgeräte, die zur Überleitung von parenteralen Lösungen oder Blut aus Glas- oder Kunst· stoffbehältern in die Blutgefäße des menschlichen Körpers dienen, sind zwecks Dosierungsmöglichkeit mit einer aus Kunststoff bestehenden Tropfkammer versehen. Die Tropfkammer besteht aus einem konisch verlaufenden Dorn mit Öffnung und der eigentlichen Tropfkammer mit dem Ausflußtubus.

Die Tropfkammer wird mittels ihres spitzen Dorns durch den Behälterverschluss hindurch eingesteckt und muss in dem Verschluss einen festen Sitz haben, damit der Dorn bei Zugbeanspruchung nicht aus dem Verschluss herausgleitet. Verschlüsse und Haftfestigkeit des Dorns sind in DIN 58 363 genormt. Infusionslösungen werden außer in Glasflaschen zum großen Teil auch in Kunststoffbehältern geliefert, die nicht mit genormten Kautschukstopfen, sondern nur mit einer einige Millimeter dicken elastischen Scheibe verschlossen sind, wodurch verstärkt die Gefahr besteht, daß der Dorn selbst bei geringer Zugbeanspruchung aus dem Behälter herausgleitet und die Infusions kontaminiert wird.

Die Dornoberfläche ist je nach dem verwendeten Kunststofftyp (Polystyrol, Polyamid oder ein anderer Kunststoff) mehr oder weniger glatt. Dies hat den Nachteil, daß der Einstechdorn keinen ausreichend festen Sitz haben kann, was insbesondere bei dünnwandigen Verschluss scheiben oder Stopfen der Fall ist.

Die Erfindung stellt sich daher die Aufgabe, einen festen Sitz des Einstechdorns und damit der Infusionsleitung in einer dünnwandigen Verschlußscheibe oder einem Stopfen zu sichern.

Gegenstand der Erfindung ist eine Tropfkammer für ein

0021405

Infusions- oder Transfusionsgerät, die einen Einstechdorn zum Einstechen in den gummiartigen, elastischen
Verschlußstopfen oder die Verschlußscheibe des Infusionsbehälters aufweist, der im Bereich seines spitz
zulaufenden Endes mit einem Einflußloch versehen ist,
dadurch gekennzeichnet, daß auf der Außenfläche des
Einstechdorns auf einem an das Einflußloch anschließenden Abschnitt in Achsrichtung parallel aufeinander folgende umfangsmässig verlaufende Profilierungen ausgebildet sind.

Durch die erfindungsgemäss vorgesehenen Profilierungen
auf der Außenfläche des Einstechdorns wird ein fester
Sitz des Einstechdorns in der Verschlußscheibe oder dem
Verschlußstopfen eines Infusionsbehälters, der z.B.
aus Glas oder Kunststoff sein kann, gewährt.

Bei einer Ausführungsform der Erfindung können die Profilierungen den Einstechdorn ringförmig umgeben.

Bei einer anderen Ausführungsform können die Profilierungen den Einstechdorn halbkreisförmig umgeben.

Die Profilierungen können durch eingebürstete, feine
parallele Rippen oder durch eingravierte parallele
Rippen oder durch eingeformte scharfkantige Rippen
gebildet sein. Die Rippen können im Querschnitt sägezahnförmig ausgestaltet sein.

Die Erfindung wird nachstehend anhand der Zeichnungen
erläutert.

Fig. 1     zeigt eine Tropfkammer.

Fig. 2 - 5 zeigen jeweils einen an einer Tropfkammer be-
           findlichenEinstechdorn sowie einen Quer-
           schnitt des Einstechdorns.

0021405

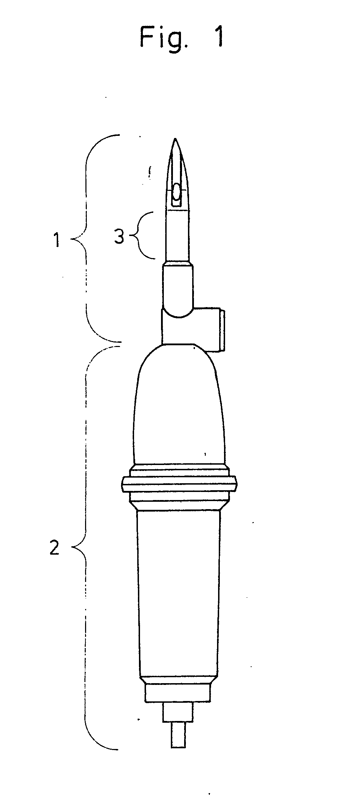

Die in Fig. 1 dargestellte Tropfkammer besteht aus einem konisch verlaufenden Einstechdorn 1 mit Lösungseinflußloch und der eigentlichen Tropfkammer 2 mit dem Ausflußtubus. Der Einstechdorn 1 wird durch den Behälterverschluss hindurch eingesteckt und muss in der Position 3 einen festen Sitz haben.

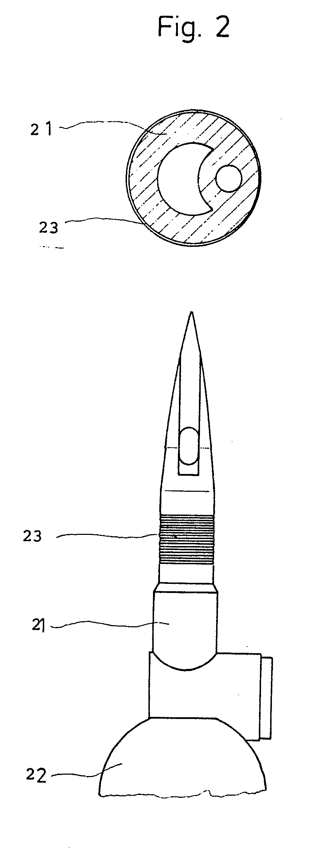

Fig. 2 zeigt den Einstechdorn 21, der mit der Tropfkammer 22 verbunden ist und der erfindungsgemäss in Achsrichtung parallel aufeinander folgende umfangsmässig verlaufende Profilierungen 23 aufweist.

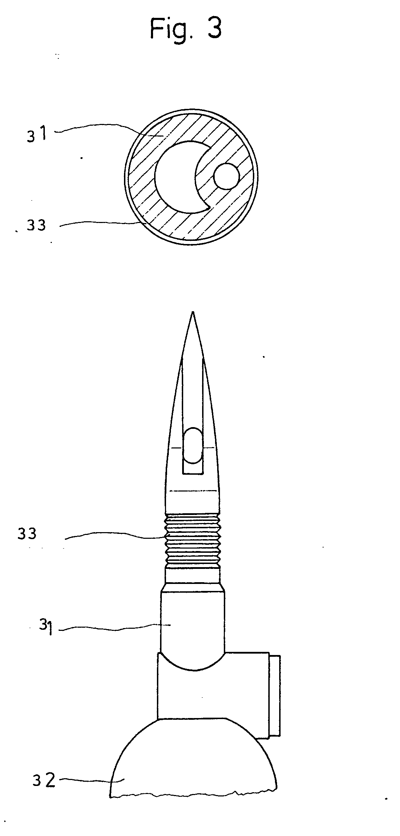

Fig. 3 zeigt einen Einstechdorn 31, die Tropfkammer 32 und die Profilierungen 33.

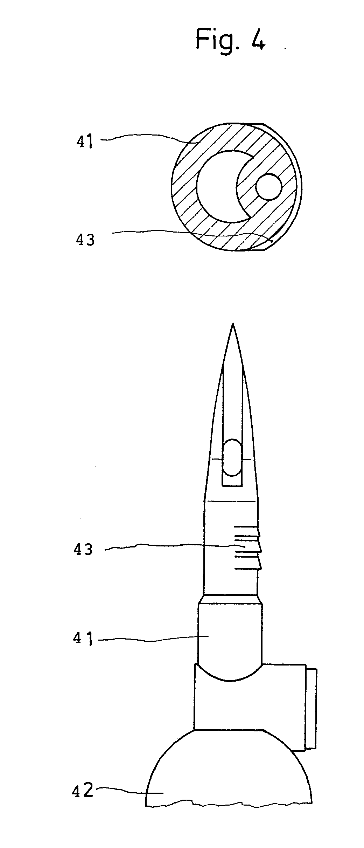

Fig. 4 zeigt den Einstechdorn 41, die Tropfkammer 42 sowie die Profilierungen 43.

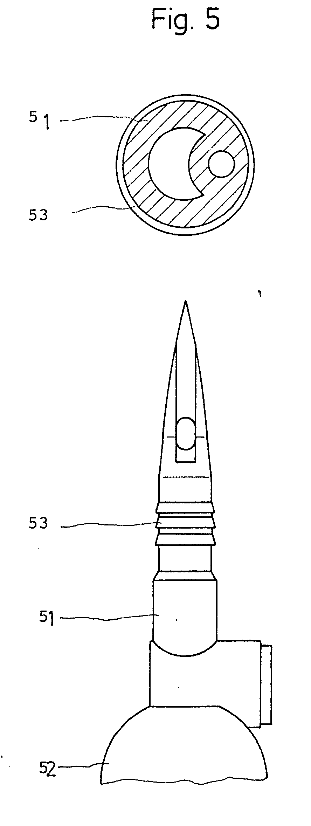

Fig. 5 zeigt den Einstechdorn 51, die Tropfkammer 52 sowie die Profilierungen 53.

Die Profilierungen gemäss Ausführungsbeispiel von Fig. 2 werden chemisch-mechanisch hergestellt, indem man die Oberfläche des Dornteils, der die Profilierungen 23 tragen soll, in rotierendem Kontakt mit einer in ein organisches Lösungsmittel, vorzugsweise Aceton, gebürsteten Bürsten- oder Pinselvorrichtung anlöst und durch Dreheinwirkung harter Borsten mit feinen, parallel verlaufenden Rillen versieht, die die Profilierungen 23 bilden. Hierdurch erhöht sich die Sitzfestigkeit des Einstechdorns von ca 12 N auf 37 N, wobei $1 \text{ N} = 1 \text{ kg m/Sek.}^2$ entspricht.

Die Profilierungen 33 gemäss Fig. 3 lassen sich technisch-mechanisch herstellen, indem man mit einer angewärmten Kombizange, in deren einer oder beiden Backen

0021405

parallel verlaufende Rillen von ca 1 mm Tiefe eingraviert sind, an dem Teil des Einstechdorns, der die Profilierungen tragen soll, bei 7o - 8o°C entsprechende Rillen einpresst. Hierdurch wird eine Auszugskraft von 6o N erzielt.

Die Profilierungen 43 gemäss Fig. 4 können durch Spritzen erzeugt werden. Wegen spezifischer Anforderungen beim Entformen von Spritzgußteilen werden sägezahnartige Ausbildungen im allgemeinen nur halbkreisförmig an dem Einstechdornteil angebracht, der die Profilierungen 43 aufweisen soll. Diese Anordnung hat den Vorteil, daß die dieAuszugskraft erhöhenden Elemente, d.h. die Profilierungen 43, in einem kostengünstigen Verfahren schon bei der Herstellung des Spritzgußteils mitgeformt werden und ein zweiter Bearbeitungsgang entfällt. Um einen möglichst hohen Auszugswiderstand zu einem elastischen Verschluss zu erhalten, sind die zahnartigen Rippen scharfkantig gestaltet.

Im Spritzverfahren erzielt man Auszugskraftwerte von 5o N bei halbkreisförmiger und von 8o N bei ringförmiger Profilierung.

Es ist jedoch auch möglich, die in Fig. 5 gezeigten scharfkantigen Profilierungen 53 unter Verwendung eines Doppelbacken-Formwerkzeugs herzustellen, so daß die scharfkantigen Absätze den ganzen Einstechdornteil auch ringförmig umgeben.

0021405

Patentansprüche

1.    Tropfkammer für ein Infusions- oder Transfusionsgerät, die einen Einstechdorn zum E instechen in den gummiartigen, elastischen Verschluss-Stopfen oder die Verschlussscheibe des Infusionsbehälters aufweist, der im Bereich
seines spitz zulaufenden Endes mit einem Einflußloch versehen ist, dadurch gekennzeichnet, daß auf der Außenfläche des Einstechdorns (1) auf einem an das Einflußloch
anschließenden Abschnitt (3) in Achsrichtung parallel aufeinander folgende umfangsmässig verlaufende Profilierungen
(23, 33, 43, 53) ausgebildet sind.

2.    Tropfkammer nach Anspruch 1, dadurch gekennzeichnet,
daß die Profilierungen der Einstechdorn ringförmig umgeben.

3.    Tropfkammer nach Anspruch 1, dadurch gekennzeichnet,
daß die Profilierungen den Einstechdorn halbkreisförmig umgeben.

4.    Tropfkammer nach Ansprüchen 1 - 3, dadurch gekennzeichnet, daß die Profilierungen durch eingebürstete
feine parallele Rippen gebildet sind.

5.    Tropfkammer nach Ansprüchen 1 - 3, dadurch gekennzeichnet, daß die Profilierungen durch eingravierte
parallele Rippen gebildet sind.

6.    Tropfkammer nach Ansprüchen 1 - 3, dadurch gekennzeichnet, daß die Profilierungen durch eingeformte
scharfkantige Rippen gebildet sind.

7.    Tropfkammer nach einem oder mehreren der vorangehenden Ansprüche, dadurch gekennzeichnet, daß die Rippen
im Querschnitt sägezahnförmig gestaltet sind.

Fig. 1

## Fig. 2

Fig. 3

## Fig. 4

41

43

43

41

42

0021405

## Fig. 5

Europäisches Patentamt

**EUROPÄISCHER RECHERCHENBERICHT**

0021405

Nummer der Anmeldung

EP 80 10 3563

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 375 870 (JOHNSON & JOHNSON)<br><br>* Abbildungen; Seite 3, Zeilen 11-31; Seite 4; Ansprüche 1, 2 *<br><br>-- | 1 |
| | DE - A - 2 221 564 (BOEHRINGER MANNHEIM GmbH)<br><br>* Abbildungen 2,3; Seite 2, Absätze 3,4; Seite 3; Seite 4, Absatz 3; Seite 5, Absätze 1-3; Ansprüche 1,4 *<br><br>-- | 1,2,7 |
| | FR - A - 1 337 891 (LAB. DR. DEBAT)<br><br>* Abbildungen 1,6; Seiten 1,2 *<br><br>----- | 1-3 |

**KLASSIFIKATION DER ANMELDUNG (Int. Cl.³)**

A 61 M 5/16

**RECHERCHIERTE SACHGEBIETE (Int. Cl.³)**

A 61 M

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-10-1980 | NOESEN |

EPA form 1503.1   06.78